(19)

**Europäisches Patentamt**
**European Patent Office**
**Office européen des brevets**

(11) **EP 4 484 544 A1**

(12) **EUROPÄISCHE PATENTANMELDUNG**

(43) Veröffentlichungstag:
**01.01.2025   Patentblatt 2025/01**

(21) Anmeldenummer: **23182766.8**

(22) Anmeldetag: **30.06.2023**

(51) Internationale Patentklassifikation (IPC):
*C12M 1/12* [(2006.01)]   *A61L 2/00* [(2006.01)]
*C12N 7/00* [(2006.01)]   *C12M 1/00* [(2006.01)]
*B01D 15/24* [(2006.01)]

(52) Gemeinsame Patentklassifikation (CPC):
**C12M 25/10; A61L 2/0088;** B01D 15/24

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Sartorius Stedim Biotech GmbH**
**37079 Göttingen (DE)**

(72) Erfinder: **Deuse, Mario**
**37079 Göttingen (DE)**

(74) Vertreter: **Gottschald**
**Patentanwälte Partnerschaft mbB**
**Klaus-Bungert-Straße 1**
**40468 Düsseldorf (DE)**

(54) **VORRICHTUNG ZUR KONTINUIERLICHEN VIRENINAKTIVIERUNG**

(57)     Die Erfindung betrifft eine Vorrichtung zur kontinuierlichen Vireninaktivierung während eines Proteinproduktionsprozesses, insbesondere eines Antikörperproduktionsprozesses, wobei die Vorrichtung eine sich in einer Axialrichtung erstreckende Hohlkörperhalteeinrichtung (1) zum Halten eines länglichen, elastisch verformbaren Hohlkörpers (2), insbesondere eines Schlauchs, aufweist, wobei die Vorrichtung mindestens zwei sich in der Axialrichtung erstreckende Rollen (3, 4), insbesondere drei oder vier Rollen (3, 4, 5, 6), aufweist, mit denen im bestimmungsgemäß montierten Zustand der Vorrichtung der Hohlkörper (2) jeweils gequetscht wird und dadurch das innere Volumen (7) des Hohlkörpers (2) in fluidtechnisch voneinander getrennte Volumenabschnitte (8) unterteilt wird, wobei die kontinuierliche Vireninaktivierung in den Volumenabschnitten (8) des Hohlkörpers (2) durchgeführt wird. Es wird vorgeschlagen, dass die Vorrichtung ein Unterstützungselement (9) aufweist, welches im bestimmungsgemäß montierten Zustand der Vorrichtung die Mantelflächen aller Rollen (3, 4, 5, 6) jeweils in mindestens einem Punkt abstützt.

Fig. 1

EP 4 484 544 A1

**Beschreibung**

Die Erfindung betrifft eine Vorrichtung zur kontinuierlichen Vireninaktivierung gemäß dem Oberbegriff von Anspruch 1, ein Verfahren zur kontinuierlichen Vireninaktivierung unter Verwendung einer vorschlagsgemäßen Vorrichtung gemäß Anspruch 17, die Verwendung einer vorschlagsgemäßen Vorrichtung zur kontinuierlichen Vireninaktivierung gemäß Anspruch 19, die Verwendung eines vormontierten Verweilzeitprodukts gemäß Anspruch 20 sowie eine Vorrichtung zur kontinuierlichen Vireninaktivierung gemäß Anspruch 22.

Die in Rede stehende Vorrichtung zur kontinuierlichen Vireninaktivierung während eines Proteinproduktionsprozesses, insbesondere eines Antikörperproduktionsprozesses, findet Anwendung im Rahmen eines Bioprozesses, insbesondere eines Downstream-Prozesses, zur Herstellung und/oder zur Qualitätskontrolle biopharmazeutischer Bioprodukte, insbesondere Proteine, Vektoren, Zellen und dergleichen, wodurch ein Produktstrom für eine nachgeschaltete Einheit, insbesondere nachgeschaltete Chromatographieeinheit, bereitgestellt werden kann.

Der Begriff "Bioprozess" meint hier biotechnologische und biopharmazeutische Prozesse, die in die Herstellung therapeutischer Bioprodukte involviert sind, wie beispielsweise Impfstoffe, Biologics, Komponenten für Zell- oder Gentherapie, oder nicht-therapeutischer Bioprodukte wie Pigmente, Biotreibstoffe oder Nährstoffe. Solche Bioprodukte können entweder durch lebende Zellen produziert werden oder die Zelle selbst kann das Bioprodukt darstellen oder das Bioprodukt kann das Resultat einer zellfreien Produktion sein, welche auf Zellkomponenten basiert, die entweder natürlichen oder nicht-natürlichen Ursprungs sind.

"Downstream-Prozess" bezeichnet in der Verfahrenstechnik alle Verfahren, die zur Abtrennung und Reinigung von Fermentationsprodukten aus einer Fermentationsbrühe eines biotechnologischen Prozesses angewandt werden. Dieser Begriff umfasst mechanische, thermische, elektrische und physiko-chemische Verfahren.

In der biopharmazeutischen Industrie geht der Trend in Richtung Intensivierung von Prozessen, wodurch immer häufiger kontinuierliche Prozessschritte implementiert werden. Bei einer Batch-Produktion, wie sie routinemäßig und in standardisierter Form durchgeführt wird, durchläuft das Produkt satzweise einen Prozessschritt nach dem anderen und wird nach jedem dieser Schritte in Lagerbehältern gesammelt. Diese Vorgehensweise kann sowohl die Nutzung der Bioprozessanordnung als auch die Produktivität des Prozesses einschränken. Eine kontinuierliche Prozessführung bedeutet hingegen, dass das Produkt mehrere Herstellungsschritte ohne Unterbrechung durchläuft. Kontinuierliche Verfahren ermöglichen es somit, die einzelnen Arbeitsschritte in deutlich kleinerem Maßstab durchzuführen, sodass sich Verbrauchsmaterialien, wie Puffer, Lösungsmittel und dergleichen, einsparen und selbst größere Prozesse im Einweg-Format durchführen lassen. Die geringere Anzahl und Größe an Lagerbehältern führt zudem zu einer kürzeren Verweildauer des Produkts im Bioprozess.

Die Vorteile einer kontinuierlichen Herstellung liegen insbesondere in einer bisher nicht erreichten Kontrolle der Produktqualität und einer besseren Agilität und Flexibilität im Umgang mit den Produktanforderungen. Ein weiterer Vorteil ist das aufgrund der kleineren Anlagenauslegung erheblich verringerte Scale-Up-Risiko. Die kontinuierliche Herstellung birgt folglich insgesamt ein großes Potenzial zur Steigerung der Wirtschaftlichkeit bei Bioprozessen.

Im Rahmen der Prozessintensivierung werden zunehmend neue Verfahren wie Einweg-Zentrifugation, Tangential Flow Filtration (TFF) oder Rapid Cycling Chromatography (RCC) entwickelt, die alle darauf abzielen, einen kontinuierlichen Produktstrom zu erzielen und damit die im Downstream-Prozess angewendeten Prozessschritte möglichst raum- und kostensparend zu gestalten.

Im Anschluss an einen ersten Chromatographieschritt, insbesondere einen Protein-A-Affinitätschromatographieschritt, erfolgt regelmäßig ein erster Virusinaktivierungsschritt. Die Implementierung eines kontinuierlichen Virusinaktivierungsschritts stellt eine Herausforderung für die Prozessentwicklung im Rahmen der Prozessintensivierung dar.

Die bekannte Vorrichtung zur kontinuierlichen Vireninaktivierung (WO 2018/208447 A1), von der die Erfindung ausgeht, dient zur kontinuierlichen Inaktivierung von Viren während eines Proteinproduktionsprozesses. Dort wird eine enge Verweilzeitverteilung des Produkts in der Vorrichtung erreicht, indem der Produktstrom in definierte Volumenabschnitte unterteilt wird. Dadurch können die einzelnen Volumenabschnitte nicht miteinander vermischt werden, wodurch deren vordefinierte Bedingungen erhalten bleiben. Des Weiteren kann durch die so hergestellte enge Verweilzeitverteilung die Inkubationszeit zur vollständigen Vireninaktivierung verkürzt werden, was insgesamt das Produkt schont.

Der konstruktive Aufbau der bekannten Vorrichtung zur kontinuierlichen Vireninaktivierung gewährleistet grundsätzlich eine universelle Einsetzbarkeit. Allerdings sorgt die konstruktive Auslegung der bekannten Vorrichtung dafür, dass lediglich ein relativ kurzer Hohlkörper mit der Vorrichtung verwendet werden kann. Dies ist darauf zurückzuführen, dass für eine längere Auslegung des Hohlkörpers ebenfalls eine längere Hohlkörperhalteeinrichtung notwendig wäre. Würde die Hohlkörperhalteeinrichtung jedoch länger ausgelegt, müssten auch die Rollen entsprechend länger ausgelegt werden. Bei einer längeren Auslegung der Rollen besteht aber das Risiko, dass sich diese durchbiegen, sodass der Abstand der Rollenoberfläche zur Hohlkörperhalteeinrichtung über die Länge der Rollen variiert. Dadurch wäre die zur bestimmungsgemäßen Funktion notwendige dichte Abtrennung der einzelnen Volumenabschnitte im Hohlkörper nicht mehr ausreichend gewährleistet. Daher werden bisher immer nur relativ kurze Vorrichtungen eingesetzt, mit denen lediglich ein relativ geringes Maximalvolumen inaktiviert werden kann, wodurch sich die Prozesszeit insgesamt verlängert sowie das restliche zu inaktivierende Produkt zwischengelagert werden muss. Dies kann wiederum problematisch werden, da Produkte aus Bioprozessen, die grundsätzlich empfindlich sind, wie zum Beispiel Antikörper, aufgrund einer zu langen Lagerzeit bzw. Prozesszeit zerstört werden könnten.

Im Ergebnis sorgt die bekannte Vorrichtung für eine lediglich limitierte Effizienz bezüglich der inaktivierbaren Volumina, der langen, das Produkt gefährdenden Prozesszeiten sowie bezüglich der Fertigungsflächennutzung.

Der Erfindung liegt das Problem zugrunde, die bekannte Vorrichtung zur kontinuierlichen Vireninaktivierung derart auszugestalten und weiterzubilden, dass deren Anwendbarkeit verbessert wird.

Das obige Problem wird bei einer Vorrichtung zur kontinuierlichen Vireninaktivierung gemäß dem Oberbegriff von Anspruch 1 durch die Merkmale des kennzeichnenden Teils von Anspruch 1 gelöst.

Wesentlich ist die grundsätzliche Überlegung, dass die Vorrichtung ein Unterstützungselement aufweist, welches die Rollen zur Außenseite der Vorrichtung hin abstützt, sodass sich diese nicht oder jedenfalls nicht nennenswert durchbiegen können.

Der besondere Aufbau der vorschlagsgemäßen Vorrichtung zur kontinuierlichen Vireninaktivierung erlaubt es, diese mit einer längeren Hohlkörperhalteeinrichtung und entsprechend einem längeren Hohlkörper, insbesondere Schlauch, auszulegen, wodurch letztlich ein größeres Volumen vireninaktiviert werden kann. Des Weiteren verbessert dies die Anwendbarkeit der Vorrichtung, da eine größere Flexibilität bezüglich des zu inaktivierenden Volumens erreicht wird. Die Rollen werden durch das vorschlagsgemäße Unterstützungselement abgestützt, wodurch ein über deren Länge gleichmäßiger Anpressdruck gewährleistet wird und die definierten Volumenabschnitte zur Erfüllung der bestimmungsgemäßen Funktion fluidtechnisch optimal voneinander getrennt bleiben. Es wird dadurch verhindert, dass sich die einzelnen Volumenabschnitte mit vor- und/oder nachgelagerten Volumenabschnitten vermischen, wodurch die Schärfe der Trennung herabgesetzt würde sowie die Gefahr einer Verfälschung der vireninaktivierenden Bedingungen bestünde.

Im Einzelnen wird vorgeschlagen, dass die Vorrichtung ein Unterstützungselement aufweist, welches im bestimmungsgemäß montierten Zustand der Vorrichtung die Mantelflächen aller Rollen jeweils in mindestens einem Punkt abstützt.

Nach den bevorzugten Ausgestaltungen nach Anspruch 2 und 3, weist die Vorrichtung eine Umlaufräderanordnung mit einem oder mehreren Sonnenrädern, Planetenrädern und Hohlrädern für eine formschlüssige Verbindung zwischen der Hohlkörperhalteeinrichtung und den Rollen auf. Durch diese Auslegung der Vorrichtung nach Vorbild eines Planetengetriebes wird ein gleichmäßiger Formschluss unter Gewährleistung einer hohen Laufruhe auf einfache Weise ermöglicht.

Die bevorzugte Ausgestaltung gemäß Anspruch 4 betrifft Details bezüglich des Zusammenwirkens und des Antriebs der Sonnenräder, Planetenräder und Hohlräder der mindestens einen Umlaufräderanordnung. Gemäß einer bevorzugten Ausgestaltung sind die Sonnenräder starr, also feststehend, ausgebildet.

Die bevorzugte Ausgestaltung gemäß Anspruch 5 betrifft Details des vorschlagsgemäßen Unterstützungselements. Diese besonderen Ausgestaltungsmöglichkeiten bieten eine besondere Flexibilität in der Anwendbarkeit der Vorrichtung.

Anspruch 6 betrifft besonders bevorzugte Ausgestaltungen der Rollen, der Hohlräder und des Unterstützungselements sowie deren formschlüssige Verbindung. Diese bevorzugten Ausgestaltungen bieten den Vorteil, dass eine Zahnverbindung hergestellt wird, wobei die entsprechenden Teilkreis- sowie Wälzkreisdurchmesser derart aufeinander abgestimmt sind, dass die beteiligten Komponenten perfekt ineinandergreifen.

Nach einer besonders bevorzugten Ausgestaltung gemäß Anspruch 7 weist die Hohlkörperhalteeinrichtung Durchführungsöffnungen auf. Diese bieten den Vorteil, dass der Hohlkörper auf einfache Weise flexibel an der Hohlkörperhalteeinrichtung anordenbar ist, insbesondere auch für den Fall, dass unterschiedlich lange Hohlkörper mit der Vorrichtung verwendet werden.

Die Ansprüche 8 und 9 betreffen besondere konstruktive Ausgestaltungen der Vorrichtung, u.a. die bevorzugte Anordnung des Hohlkörpers an der Hohlkörperhalteeinrichtung. Diese Ausgestaltungen bieten einerseits den Vorteil, dass die bestimmungsgemäße Funktion auf besonders einfache Weise herstellbar ist und andererseits, dass die Vorrichtung eine besondere Flexibilität bezüglich der Anwendbarkeit bietet.

Die Ansprüche 10 bis 13 betreffen ebenfalls besondere konstruktive Ausgestaltungen, wie die bevorzugte Rollenanzahl, deren Zusammenspiel mit der Hohlkörperhalteeinrichtung zur Herstellung einer Fluid-Förderung gemäß dem Prinzip einer Verdrängerpumpe sowie die bevorzugte Anordnung an einer Tragstruktur. Diese Ausgestaltungen bieten den Vorteil, dass durch eine größere Anzahl an Rollen mehr Volumenabschnitte im Hohlkörper erzeugt werden können, wodurch eine schärfere Trennung während der Fluid-Förderung ermöglicht wird. Nach einer besonders bevorzugten Ausgestaltung entspricht der Abstand zwischen Hohlkörperhalteeinrichtung und Rollen höchstens der zweifachen Wandstärke des Hohlkörpers, wodurch eine Dichtigkeit auf einfache und zuverlässige Weise hergestellt wird, was die für die bestimmungsgemäße Funktion notwendige fluidtechnische Dichtigkeit der vorschlagsgemäßen Vorrichtung garantiert. Die bevorzugte Anordnung der Vorrichtung an einer Tragstruktur bietet den Vorteil einer besonders kompakten und bauraumsparenden Bauweise.

Eine besonders bevorzugte Ausgestaltung gemäß Anspruch 14 definiert, dass die Vorrichtung vorzugsweise als Einwegkomponente ausgestaltet ist. Diese Ausgestaltung ist insbesondere in einem Bioprozess besonders vorteilhaft, da somit die notwendige Sterilität sichergestellt werden kann. Dies ist insbesondere vorteilhaft, da ein Nutzer somit den Hohlkörper und/oder die Hohlkörperhalteeinrichtung nach Benutzung auf besonders einfache Art und Weise für einen neuen Prozess ersetzen kann.

Eine weiter bevorzugte Ausgestaltung gemäß Anspruch 15 betrifft die Ausgestaltung der zur bestimmungsgemäßen Funktion mindestens notwendigen Bestandteile der Vorrichtung als eine Baugruppe. Dies bietet den Vorteil einer erhöhten Flexibilität bezüglich der Anwendbarkeit der vorschlagsgemäßen Vorrichtung, da der Nutzer auf einfache Art beispielsweise kontaminierte Baugruppen austauschen kann.

Die weiter bevorzugte Ausgestaltung gemäß Anspruch 16 definiert für einzelne Bauteile der Vorrichtung die bevorzugte Herstellungsweise. Dadurch ist die Vorrichtung auf besonders einfache und effiziente Weise herstellbar.

Nach einer weiteren Lehre gemäß Anspruch 17, der eigenständige Bedeutung zukommt, wird ein Verfahren zur kontinuierlichen Vireninaktivierung während eines Proteinproduktionsprozesses, insbesondere eines Antikörperproduktionsprozesses, unter Verwendung einer vorschlagsgemäßen Vorrichtung beansprucht.

Nach einer bevorzugten Ausgestaltung gemäß Anspruch 18 wird das vorschlagsgemäße Verfahren in Kombination mit Chromatographieverfahren und/oder mit Filtrationsverfahren durchgeführt. Dies bietet die Möglichkeit der einfachen Einbindung des vorschlagsgemäßen Verfahrens in einen bereits bestehenden Bioprozess. Auf alle Ausführungen zur vorschlagsgemäßen Vorrichtung darf insoweit verwiesen werden.

Nach einer weiteren Lehre gemäß Anspruch 19, der eigenständige Bedeutung zukommt, wird die Verwendung einer Vorrichtung zur Umsetzung eines Verfahrens zur kontinuierlichen Vireninaktivierung während eines Proteinproduktionsprozesses, insbesondere eines Antikörperproduktionsprozesses, beansprucht. Auf alle Ausführungen zur vorschlagsgemäßen Vorrichtung und zum vorschlagsgemäßen Verfahren darf insoweit verwiesen werden.

Nach einer weiteren Lehre gemäß Anspruch 20, der eigenständige Bedeutung zukommt, wird die Verwendung eines vormontierten Verweilzeitprodukts zur kontinuierlichen Vireninaktivierung während eines Proteinproduktionsprozesses beansprucht. Die, zumindest teilweise, Vormontage der Vorrichtung macht es besonders einfach, diese schnell betriebsbereit zu machen. Die daraus resultierende Austauschbarkeit der Vorrichtung als Ganzes oder von Teilen davon macht es zudem besonders einfach, sterile Bedingungen zu schaffen und/oder aufrechtzuerhalten.

Gemäß Anspruch 21 ist das vormontierte Verweilzeitprodukt vorzugsweise als Einwegkomponente ausgestaltet, sodass ein kosteneffizienter Prozess und gleichzeitig perfekte, sterile Bedingungen gewährleistet sind. Auch für diese vierte Lehre gelten alle Ausführungen zur ersten, zweiten und dritten Lehre in vollem Umfang.

Nach einer weiteren Lehre gemäß Anspruch 22, der eigenständige Bedeutung zukommt, wird eine Vorrichtung zur kontinuierlichen Vireninaktivierung während eines Proteinproduktionsprozesses, insbesondere eines Antikörperproduktionsprozesses, beansprucht, wobei die Vorrichtung eine sich in einer Axialrichtung erstreckende Hohlkörperhalteeinrichtung zum Halten eines länglichen, elastisch verformbaren Hohlkörpers, insbesondere eines Schlauchs, aufweist, wobei die Vorrichtung mindestens zwei sich in der Axialrichtung erstreckende Rollen, insbesondere drei oder vier Rollen, aufweist, mit denen im bestimmungsgemäß montierten Zustand der Vorrichtung der Hohlkörper jeweils gequetscht wird und dadurch das innere Volumen des Hohlkörpers in fluidtechnisch voneinander getrennte Volumenabschnitte unterteilt wird, wobei die kontinuierliche Vireninaktivierung in den Volumenabschnitten des Hohlkörpers durchgeführt wird. Auch für diese fünfte Lehre gelten alle Ausführungen zur ersten, zweiten, dritten und vierten Lehre in vollem Umfang.

Im Einzelnen wird dabei vorgeschlagen, dass die Vorrichtung zum Antrieb der Rollen mindestens eine Umlauffräderanordnung mit jeweils einem Sonnenrad, mehreren mit diesem in Eingriff stehenden Planetenrädern und einem mit diesen in Eingriff stehenden Hohlrad aufweist.

Im Folgenden wird die Erfindung anhand einer lediglich ein Ausführungsbeispiel darstellenden Zeichnung näher erläutert. In der Zeichnung zeigt

Fig. 1 ein Ausführungsbeispiel einer vorschlagsgemäßen Vorrichtung zur kontinuierlichen Vireninaktivierung in einer perspektivischen Darstellung,

Fig. 2 eine vorschlagsgemäße Vorrichtung gemäß Fig. 1 in einer Schnittdarstellung a) der formschlüssigen Verbindung zwischen Sonnenrad, Planetenrädern und Hohlrad und b) der formschlüssigen Verbindung zwischen Unterstützungselement und Rollen,

Fig. 3 eine vorschlagsgemäße Vorrichtung gemäß Fig. 1 in einer perspektivischen Darstellung und in einer Schnittdarstellung, welche die bevorzugte Anordnung des Hohlkörpers darstellt,

Fig. 4 ein Ausführungsbeispiel eines vorschlagsgemäßen Verweilzeitprodukts zur kontinuierlichen Vireninaktivierung für eine vorschlagsgemäße Vorrichtung gemäß Fig. 1 in einer perspektivischen Darstellung.

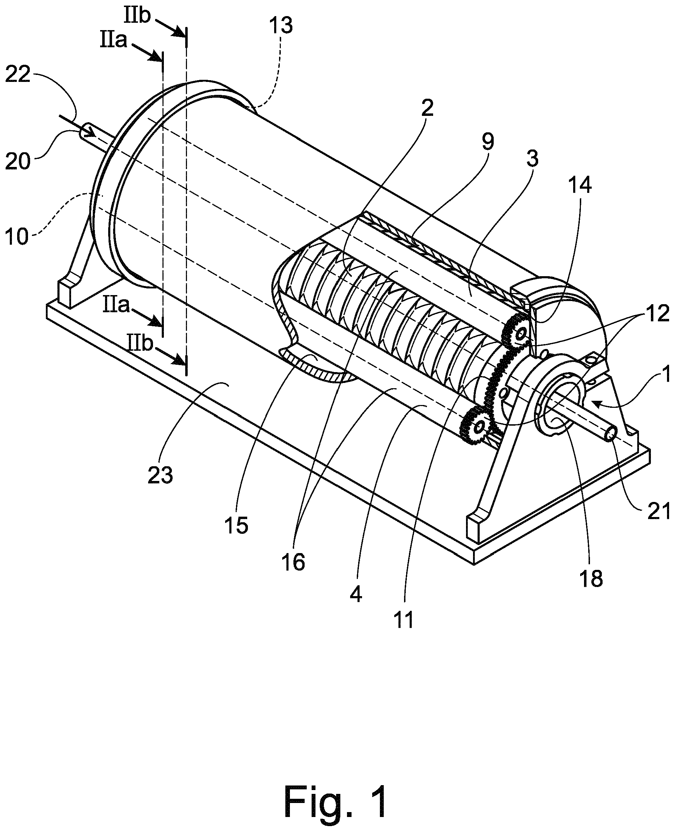

In Fig. 1 ist eine vorschlagsgemäße Vorrichtung zur kontinuierlichen Vireninaktivierung während eines Proteinproduktionsprozesses, insbesondere eines Antikörperproduktionsprozesses, dargestellt. Diese findet Einsatz im Downstream-Prozess eines Bioprozesses, insbesondere bei der Produktion, Aufreinigung und/oder Qualitätskontrolle biopharmazeutischer Produkte, wie beispielsweise während der Produktion eines Proteins. Solche Proteine können Wachstumsfaktoren, Hormone, Enzyme und insbesondere Antikörper, Antikörperderivate oder dergleichen sein. Das vorschlagsgemäße Verfahren kann dazu verwendet werden, sicherzustellen, dass ein biopharmazeutisches Produkt keine aktiven Viruspartikel jeglichen Typs oberhalb eines durch beispielsweise den Hersteller und/oder Zulassungsbehörden, etc. festgelegten, bestimmten Schwellenwerts, insbesondere überhaupt keine aktiven Viruspartikel, enthält.

Das Zielprotein kann dabei entweder direkt oder indirekt aus einem Bioreaktor stammen, insbesondere nachdem prozessierende Schritte, insbesondere Schritte des Downstream-Prozesses, wie Filtration, Präzipitation, und/oder chromatographische Auftrennungsschritte oder dergleichen, durchgeführt wurden. Solche chromatographischen Schritte können beispielsweise affinitätschromatographische Schritte sein, insbesondere affinitätschromatographische Schritte unter Verwendung von Protein A.

Die Vorrichtung weist eine sich in einer Axialrichtung erstreckende Hohlkörperhalteeinrichtung 1 zum Halten eines länglichen, elastisch verformbaren Hohlkörpers 2, insbesondere eines Schlauchs, auf.

Der Begriff "Hohlkörperhalteeinrichtung" ist vorliegend weit zu verstehen und meint jede zylindrisch, insbesondere hohlzylindrisch, ausgestaltete Vorrichtung zum Halten eines Hohlkörpers.

Der Begriff "Hohlkörper" ist vorliegend weit zu verstehen und meint jede Art von fluiddichtem, insbesondere biegeschlaffem, Körper, der innen einen Kanal für die Durchleitung eines Fluids ausbildet, insbesondere einen Schlauch oder dergleichen. Vorschlagsgemäße Hohlkörper sind vorzugsweise aus Naturkautschuk, synthetischen Kautschuken oder Kunststoffen, wie PVC, PUR, PA, PE oder dergleichen, gefertigt.

Vorzugsweise ist der Hohlkörper 2 im Querschnitt rund, oval, rechteckig oder dergleichen ausgestaltet und weist einen äußeren Durchmesser von vorzugsweise 5 mm bis 50 mm, weiter vorzugsweise von 7,5 mm bis 15 mm, auf, mit einer bevorzugten Wandstärke von 0,5 mm bis 10 mm, weiter vorzugsweise von 1 bis 5 mm. Gemäß einer besonders bevorzugten Ausgestaltung beträgt der äu-ßere Durchmesser des Hohlkörpers 2 10 mm mit einer Wandstärke von 1 mm. Die Vorrichtung weist mindestens zwei sich in der Axialrichtung erstreckende Rollen 3, 4, insbesondere drei oder vier Rollen 3, 4, 5, 6, auf, mit denen im bestimmungsgemäß montierten Zustand der Vorrichtung der Hohlkörper 2 jeweils gequetscht wird und dadurch das innere Volumen 7 des Hohlkörpers 2 in fluidtechnisch voneinander getrennte Volumenabschnitte 8 unterteilt wird. Die kontinuierliche Vireninaktivierung wird in den Volumenabschnitten 8 des Hohlkörpers 2 durchgeführt.

Der Begriff "Rolle" ist vorliegend weit zu verstehen und meint jede Art von Zylindern, Hohlzylindern, Walzen, Stangen oder dergleichen, welche als Grundfläche im Wesentlichen einen Kreis aufweisen.

Wesentlich bei der vorschlagsgemäßen Vorrichtung ist nun, dass die Vorrichtung ein Unterstützungselement 9 aufweist, welches im bestimmungsgemäß montierten Zustand der Vorrichtung die Mantelflächen aller Rollen 3, 4, 5, 6 jeweils in mindestens einem Punkt abstützt.

Der Begriff "Mantelfläche" meint vorliegend jene Oberfläche, welche sich aus dem Produkt des Umfangs und der Höhe ergibt.

Es ist besonders bevorzugt, dass die Hohlkörperhalteeinrichtung 1 ein Längen zu Durchmesser Verhältnis von mindestens 2:1 aufweist. Zusätzlich oder alternativ ist besonders bevorzugt, dass die Hohlkörperhalteeinrichtung 1 und/oder die Rollen 3, 4, 5, 6 biegesteif ausgestaltet sind.

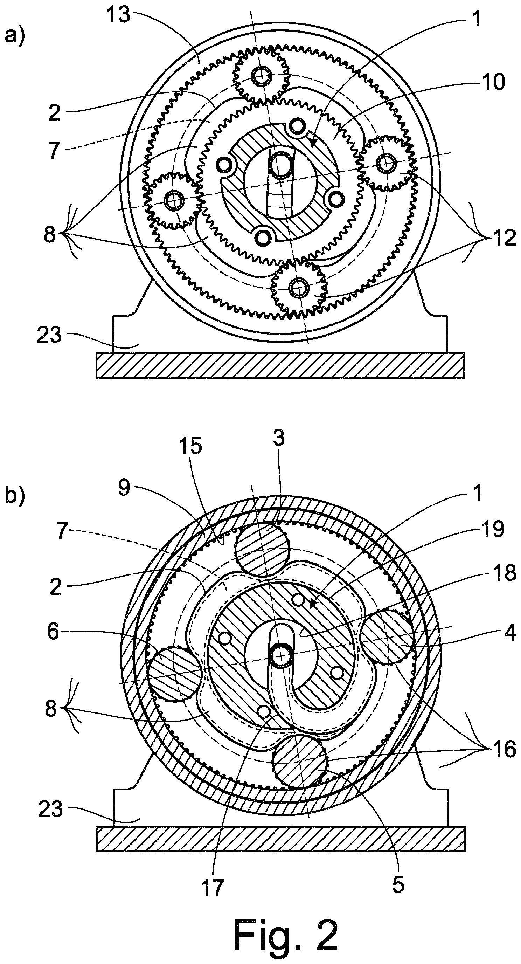

Hier und vorzugsweise, wie in Fig. 1 und 2 dargestellt, weist die Vorrichtung genau ein Unterstützungselement 9 auf. Weiter vorzugsweise weist die Vorrichtung mehrere Unterstützungselemente 9 auf. Das Unterstützungselement 9 umschließt vorzugsweise im bestimmungsgemäßen Zustand mindestens eine der Rollen 3, 4, 5, 6, vorzugsweise alle Rollen 3, 4, 5, 6, derart, dass das Unterstützungselement 9 mindestens eine der Rollen 3, 4, 5, 6, vorzugsweise alle Rollen 3, 4, 5, 6, in mindestens einem Punkt formschlüssig abstützt. Vorzugsweise stützt das Unterstützungselement mindestens eine der Rollen 3, 4, 5, 6, vorzugsweise alle Rollen 3, 4, 5, 6, weiter vorzugsweise die Mantelflächen aller Rollen 3, 4, 5, 6, zumindest über deren mittleren Abschnitt, weiter vorzugsweise über deren gesamte Länge, formschlüssig ab. Vorzugsweise weist die Hohlkörperhalteeinrichtung 1 ein Längen zu Durchmesser Verhältnis von mindestens 3:1, weiter vorzugsweise von mindestens 4:1, weiter vorzugsweise von mindestens 6:1, weiter vorzugsweise von mindestens 10:1, auf.

Hier und vorzugsweise ist die Vorrichtung derart ausgelegt, dass durch den Formschluss zwischen Rollen 3, 4, 5, 6 und Unterstützungselement 9 die Rollen 3, 4, 5, 6 den Hohlkörper 2 derart vollständig abdrücken, dass eine fluidtechnische Verbindung unterbrochen wird. Dadurch wird ein für die bestimmungsgemäße Funktion notwendiger Eingriff zwischen Rollen 3, 4, 5, 6 und Hohlkörper 2 und somit schließlich die Dichtigkeit der einzelnen Volumenabschnitte 8 gewährleistet. Die Volumenabschnitte 8 sind vorzugsweise durch eine entsprechende Auswahl der Rollenanzahl möglichst klein ausgelegt, um eine schärfere Trennung mindestens eines Vireninaktivierungsparameters, insbesondere des pH, zu ermöglichen. Das Abdrücken des Hohlkörpers 2 bewirkt jedoch eine Verringerung des zur Verfügung stehenden inneren Hohlkörpervolumens 7. Dieser Verringerung wird durch die bevorzugte Auslegung der Hohlkörperhalteeinrichtung 1 mit einem Längen zu Durchmesser Verhältnis von mindestens 2:1 entgegengewirkt, da dadurch ein längerer Hohlkörper 2 an der Hohlkörperhalteeinrichtung 1 anordenbar ist und folglich ein größeres Volumen 7 im Hohlkörper 2 für eine Vireninaktivierung zur Verfügung steht.

Hier und vorzugsweise weist die Vorrichtung zum Antrieb der Rollen 3, 4, 5, 6, mindestens eine Umlaufräderanordnung, vorzugsweise zwei oder mehr axial voneinander beabstandete Umlaufräderanordnungen, auf. Unter einer Umlaufräderanordnung ist vorliegend eine Einheit zur Übertragung von Drehbewegungen gemeint, die wie bei einem Umlaufrädergetriebe die miteinander in Eingriff stehenden Komponenten "Sonnenrad", "Planetenräder" und "Hohlrad" aufweist. Im Einzelnen weist eine Umlaufräderanordnung in diesem Sinne jeweils ein Sonnenrad 10, 11, mehrere mit diesem in Eingriff stehende Planetenräder 12 und ein mit diesen in Eingriff stehendes Hohlrad 13, 14 auf. Die Planetenräder 12 können wie bei einem Umlaufrädergetriebe in einem Planetenradträger gelagert sein, alternativ aber auch ohne einen solchen gehalten werden. Vorzugsweise stehen die Planetenräder 12 durch eine Zahnverbindung, insbesondere durch eine Evolventenverzahnung, mit einem der zugeordneten Sonnenräder 10, 11 und/oder der Hohlräder 13, 14 in kämmendem Eingriff. Gerade bei Verwendung einer Verzahnung wird vorzugsweise auf einen Planetenradträger verzichtet. Vorzugsweise weist die Hohlkörperhalteeinrichtung 1 mindestens ein, insbesondere endseitig angeordnetes, Sonnenrad 10, 11, vorzugsweise zwei axial voneinander beabstandete, insbesondere endseitig angeordnete Sonnenräder 10, 11 auf, das/die mit der Hohlkörperhalteeinrichtung 1 im Übrigen drehfest ist/sind. Zusätzlich oder alternativ ist bevorzugt, dass die Rollen 3, 4, 5, 6 jeweils mindestens ein, insbesondere endseitig angeordnetes, Planetenrad 12, vorzugsweise zwei axial voneinander beabstandete, insbesondere endseitig angeordnete, Planetenräder 12, aufweisen, das/die mit der jeweiligen Rolle 3, 4, 5, 6 im Übrigen drehfest ist/sind. Weiter ist zusätzlich oder alternativ bevorzugt, dass das Unterstützungselement 9 mindestens ein, insbesondere endseitig angeordnetes, Hohlrad 13, 14, vorzugsweise zwei axial voneinander beabstandete, insbesondere endseitig angeordnete, Hohlräder 13, 14, aufweist, das/die mit dem Unterstützungselement 9 im Übrigen drehfest ist/sind (Fig. 2a). Das jeweilige Hohlrad 13, 14 kann dabei separat von oder einstückig mit dem Unterstützungselement 9 ausgestaltet sein.

Der Begriff "endseitig" ist vorliegend weit zu verstehen und meint eine Anordnung im axialen Endbereich oder den beiden axialen Endbereichen, insbesondere an dem axialen Ende oder den axialen Enden, der jeweiligen sich in eine Axialrichtung erstreckenden, insbesondere zylindrischen, Komponente der Umlaufräderanordnung. Ein axialer Endbereich ist dabei insbesondere definiert als die letzten 15%, vorzugsweise letzten 10%, weiter vorzugsweise letzten 5%, der axialen Erstreckung der jeweiligen Komponente. "Zwei axial voneinander beabstandete, endseitig angeordnete" Sonnenräder bzw. Planetenräder bzw. Hohlräder meint, dass je eines der Räder in dem einen axialen Endbereich bzw. an dem einen axialen Ende angeordnet ist und das jeweils andere der Räder in dem anderen axialen Endbereich bzw. an dem anderen axialen Ende angeordnet ist. Zusätzlich oder alternativ zu mindestens einer der endseitig angeordneten Umlaufräderanordnungen kann grundsätzlich auch mindestens eine entsprechende Umlaufräderanordnung im Bereich axial zwischen den Endbereichen angeordnet sein. So ist es bei einer relativ großen axialen Erstreckung der Vorrichtung und entsprechend großen Länge der Rollen 3, 4, 5, 6 denkbar, eine weitere Umlaufräderanordnung axial in der Mitte des Unterstützungselements 9 oder auch mehrere Umlaufräderanordnungen in vorzugsweise gleichmäßigen axialen Abständen über die axiale Erstreckung des Unterstützungselements 9 anzuordnen, um einem Durchbiegen der Rollen 3, 4, 5, 6 entgegenzuwirken.

Vorzugsweise ist/sind im bestimmungsgemäßen Betrieb der Vorrichtung das Hohlrad 13, 14 oder mindestens eines der Planetenräder 12 mindestens einer der Umlaufräderanordnungen motorisch angetrieben, weiter vorzugsweise über einen elektrischen Antriebsmotor der Vorrichtung. Gemäß einer anderen, hier nicht dargestellten Ausgestaltung kann alternativ auch das Sonnenrad 10, 11 mindestens einer der Umlaufräderanordnungen solchermaßen angetrieben sein. Vorzugsweise sind im bestimmungsgemäßen Betrieb der Vorrichtung die Sonnenräder 10, 11 aber nicht angetrieben und insbesondere starr angeordnet.

Der Begriff "starr" meint, dass das jeweilige Sonnenrad einer Drehbewegung des Hohlrades und/oder der Planetenräder nicht folgt, sondern ortsfest, insbesondere zu einer Tragstruktur oder einem Gehäuse, ist. Die Drehbewegung eines Hohlrads bewirkt dabei ausschließlich eine Drehbewegung der Planetenräder, die sich dann auf dem nichtdrehenden, also feststehenden, Sonnenrad abrollen.

Das Unterstützungselement 9 ist hier und vorzugsweise hohlzylindrisch ausgestaltet (Fig. 1). Das Unterstützungselement 9 kann aber auch ringförmig ausgestaltet sein, wobei nur ein einzelnes ringförmiges Unterstützungselement 9 vorgesehen sein kann oder mehrere ringförmige Unterstützungselemente 9 axial nebeneinander angeordnet sein können. Auch kann das Unterstützungselement 9 spiralförmig ausgestaltet sein.

Vorzugsweise weist das Unterstützungselement 9 eine umlaufende, insbesondere zylindrische, in Umfangsrichtung und/oder Axialrichtung ebene Innenoberfläche 15 auf. Die Rollen weisen vorzugsweise eine zylindrische, in Umfangsrichtung und/oder Axialrichtung ebene Außenoberfläche 16 auf. Hier und vorzugsweise, wie in Fig. 2b dargestellt, rollt sich bestimmungsgemäßen Betrieb der Vorrichtung die Innenoberfläche 15 des Unterstützungselements 9 auf der Außenoberfläche der Rollen 16 vollständig ab. Wie in Fig. 1 dargestellt, ist das Unterstützungselement 9 vorzugsweise als axiale Verlängerung eines Hohlrads 13, 14 ausgelegt. Alternativ ist das Unterstützungselement 9 vorzugsweise als axiale Verlängerung der beiden Hohlräder 13, 14 ausgelegt, vorzugsweise derart, dass das Unterstützungselement 9 die Rollen 3, 4, 5, 6 über lediglich einen Teil deren Länge abstützt. Alternativ verbindet das Unterstützungselement 9 vorzugsweise die beiden Hohlräder 13, 14 axial miteinander. Das Unterstützungselement 9 ist vorzugsweise einstückig, das heißt integral, oder mehrstückig ausgestaltet.

Der Innendurchmesser des Unterstützungselements 9 ist vorzugsweise derart auf den Außendurchmesser der Rollen 3, 4, 5, 6 abgestimmt, dass sich die Rollen 3, 4, 5, 6 und das Unterstützungselement 9 aneinander in einem Wälzpunkt abwälzen (Fig. 2b). Zusätzlich oder alternativ sind im bestimmungsgemäß montierten Zustand der Vorrichtung die Teilkreisdurchmesser der Planetenräder 12 der Rollen 3, 4, 5, 6 mit den Wälzkreisdurchmessern der Rollen 3, 4, 5, 6 im Übrigen identisch. Zusätzlich sind vorzugsweise die Teilkreisdurchmesser der Hohlräder 13, 14 mit dem Wälzkreisdurchmesser des Unterstützungselements 9 identisch.

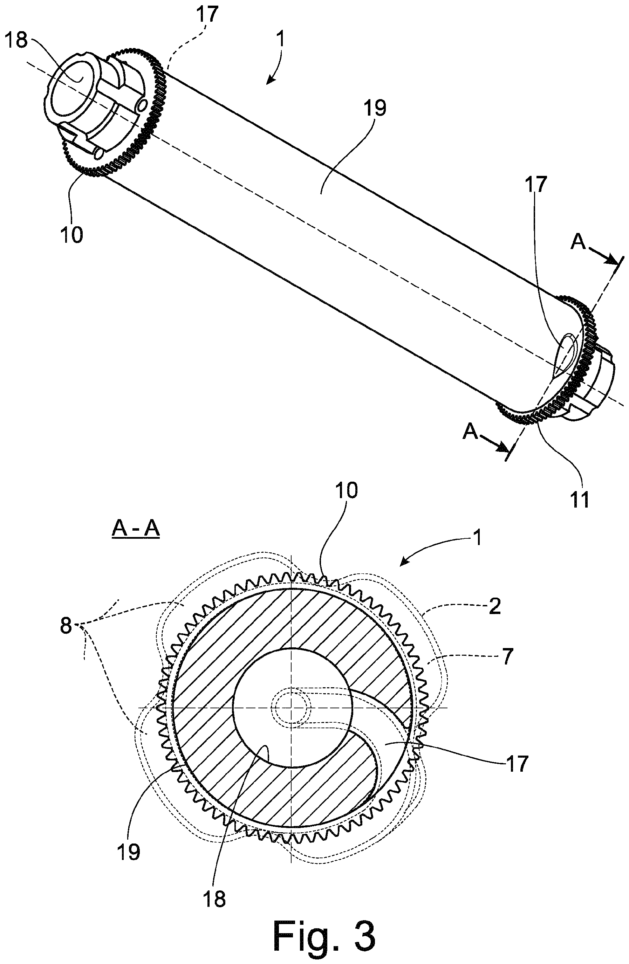

Wie in den Fig. 2a und Fig. 3 dargestellt, weist die Hohlkörperhalteeinrichtung 1 hier und vorzugsweise mindestens zwei, insbesondere mindestens drei oder vier, Durchführungsöffnungen 17 auf. Die Durchführungsöffnungen 17 verbinden vorzugsweise die Innenoberfläche 18 oder das Innere der Hohlkörperhalteeinrichtung 1 mit der Außenoberfläche 19 oder dem Äußeren der Hohlkörperhalteeinrichtung 1. Der Hohlkörper 2 ist im bestimmungsgemäß montierten Zustand der Vorrichtung durch die Durchführungsöffnungen 17 hindurchgeführt. Hier und vorzugsweise bilden die Durchführungsöffnungen 17 die einzigen Verbindungen zwischen der Innenoberfläche 18 bzw. dem Inneren der Hohlkörperhalteeinrichtung 1 und der Außenoberfläche 19 bzw. dem Äußeren der Hohlkörperhalteeinrichtung 1. Hier und vorzugsweise sind die Durchführungsöffnungen 17 derart angeordnet, dass der Hohlkörper 2 zur Herstellung des bestimmungsgemäßen Zustands der Vorrichtung auf besonders einfache Weise an der Hohlkörperhalteeinrichtung 1 montiert werden kann. Dazu wird vorzugsweise der Hohlkörper 2 zunächst durch eine erste axiale Öffnung in das Innere der Hohlkörperhalteeinrichtung 1 und dann durch die erste, vorzugsweise als radiale Öffnung ausgestaltete, Durchführungsöffnung 17 nach außen geführt. Der Hohlkörper 2 wird vorzugsweise von außen um die Hohlkörperhalteeinrichtung 1 gewickelt und anschließend durch die mindestens zweite, vorzugsweise als radiale Öffnung ausgestaltete, Durchführungsöffnung 17 wieder ins Innere der Hohlkörperhalteeinrichtung 1 geführt. Vorzugsweise wird der Hohlkörper 2 schließlich von der Innenoberfläche der Hohlkörperhalteeinrichtung 1 zentral aus der Hohlkörperhalteeinrichtung 1 durch eine zweite axiale Öffnung herausgeführt. Vorzugsweise weist die Hohlkörperhalteeinrichtung 1 in Axialrichtung nebeneinander mehrere, vorzugsweise als radiale Öffnungen ausgestaltete, Durchführungsöffnungen 17 für unterschiedliche Schlauchlängen auf.

Hier und vorzugsweise weist der Hohlkörper 2 einen Fluideinlass 20 und einen Fluidauslass 21 auf. Hier und vorzugsweise ist der Hohlkörper 2 auf die Hohlkörperhalteeinrichtung 1 aufgewickelt. Vorzugsweise ist der Hohlkörper 2 zumindest größtenteils quer, insbesondere im Wesentlichen orthogonal, zur sich in der Axialrichtung erstreckenden geometrischen Längsachse der Hohlkörperhalteeinrichtung 1 angeordnet, wie in Fig. 1 dargestellt.

Gemäß einer besonders bevorzugten Ausgestaltung ist der Hohlkörper 2 hier und vorzugsweise im bestimmungsgemäßen Betrieb der Vorrichtung derart an der Hohlkörperhalteeinrichtung 1 angeordnet, dass bei dem bestimmungsgemä-ßen Eingriff zwischen Rollen 3, 4, 5, 6 und Hohlkörper 2 kein Abstand zwischen zwei Hohlkörperwindungen existiert. Vorzugsweise wird zur Herstellung des bestimmungsgemäßen Zustands der Hohlkörper 2 an der Hohlkörperhalteeinrichtung 1 derart angeordnet, dass der Abstand zwischen zwei Hohlkörperwindungen mindestens

$$((U/2) - d)$$

entspricht (U = Hohlkörperumfang, d = Hohlkörperdurchmesser).

Im bestimmungsgemäß montierten Zustand der Vorrichtung ist hier und vorzugsweise ein Zielprotein und vireninaktivierende Bedingungen beinhaltender Flüssigkeitsstrom 22 durch den Fluideinlass 20 in die Vorrichtung einleitbar. Zum Vorsehen einer Mindestverweildauer des Flüssigkeitsstroms 22 in der Vorrichtung ist der Flüssigkeitsstrom 22 vorzugsweise durch die Vorrichtung hindurchleitbar und anschließend durch den Fluidauslass 21 aus der Vorrichtung ausleitbar. Die Vorrichtung ist vorzugsweise beidseitig durchströmbar, derart, dass der Flüssigkeitsstrom 22 alternativ durch den Fluidauslass 21 in die Vorrichtung einleitbar und durch den Fluideinlass 20 aus der Vorrichtung ausleitbar ist. Gemäß einer besonders bevorzugten Ausgestaltung können die Länge und der Durchmesser des Hohlkörpers 2 sowie die Flussrate des Flüssigkeitsstroms 22, vorzugsweise von einem Nutzer, derart eingestellt werden, dass eine bestimmte Verweildauer des Flüssigkeitsstroms 22 in der Vorrichtung erzielt werden kann. Daraus ergibt sich, dass die Möglichkeit der längeren Auslegung des Hohlkörpers mit einer erhöhten Flexibilität in der Anwendbarkeit der vorschlagsgemäßen Vorrichtung einhergeht.

Bei gerader Anzahl der Rollen 3, 4, 5, 6 sind diese hier und vorzugsweise am Außenumfang der Hohlkörperhalteeinrichtung 1 gegenüberliegend, insbesondere gleichmäßig über den Außenumfang der Hohlkörperhalteeinrichtung 1 verteilt, angeordnet. Auch bei ungerader Anzahl sind diese vorzugsweise gleichmäßig über den Außenumfang der Hohlkörperhalteeinrichtung 1 verteilt angeordnet.

Im bestimmungsgemäßen Betrieb der Vorrichtung wird vorzugsweise durch eine Relativbewegung der Rollen 3, 4, 5, 6 und der Hohlkörperhalteeinrichtung 1 zueinander eine Fluid-Förderung im Hohlkörper 2 gemäß dem Prinzip einer Verdrängerpumpe derart erzeugt, dass die voneinander getrennten Volumenabschnitte 8 im Hohlkörper 2 im bestimmungsgemäßen Betrieb der Vorrichtung mit den voreingestellten vireninaktivierenden Bedingungen über die Prozesslaufzeit zueinander unvermischt transportiert werden.

Vorzugsweise entspricht im bestimmungsgemäß montierten Zustand der Vorrichtung der Abstand zwischen Hohlkörperhalteeinrichtung 1 und Rollen 3, 4, 5, 6 höchstens der zweifachen Wandstärke des Hohlkörpers 2.

Wie in den Fig. 1, Fig. 2a und Fig. 2b dargestellt, weist die Vorrichtung hier und vorzugsweise eine Tragstruktur 23 auf. Die Tragstruktur 23 weist hier und vorzugsweise eine Standplatte und darauf ein Gestell auf. Vorzugsweise sind alle Bestandteile der Vorrichtung direkt oder indirekt an der Tragstruktur 23 angeordnet. Gemäß einer ebenfalls bevorzugten Ausgestaltung ist zumindest die Hohlkörperhalteeinrichtung 1 direkt an der Tragstruktur 23 angeordnet.

Hier und vorzugsweise sind alle Bestandteile der Vorrichtung, vorzugsweise zumindest der Hohlkörper 2 und/oder die Hohlkörperhalteeinrichtung 1, als Einwegkomponente ausgestaltet. Die Einwegkomponente ist vorzugsweise zumindest teilweise aus Kunststoff gefertigt, sodass sie mit geringem Aufwand realisiert werden kann. In besonders bevorzugter Ausgestaltung ist mindestens eine dieser Komponenten zumindest zum Teil, vorzugsweise überwiegend, aus einem Kunststoffmaterial ausgelegt, vorzugsweise derart, dass die Einwegkomponente zumindest teilweise aus einem Silikonmaterial und/oder Polymermaterial und/oder Biokunststoff hergestellt ist.

Die zur bestimmungsgemäßen Funktion mindestens notwendigen Bestandteile der Vorrichtung, insbesondere zumindest umfassend die Hohlkörperhalteeinrichtung 1 und den Hohlkörper 2, bilden vorzugsweise eine Baugruppe. Zusätzlich oder alternativ bilden vorzugsweise zumindest die Tragstruktur 23, die Rollen 3, 4, 5, 6 und das Unterstützungselement 9 eine Baugruppe, die als vormontierte oder einstückige Einheit ausgestaltet ist.

Hier und vorzugsweise ist/sind die Tragstruktur 23, die Hohlkörperhalteeinrichtung 1, die Rollen 3, 4, 5, 6 und/oder das Unterstützungselement 9, insbesondere einzeln oder zusammen, im Kunststoff-Spritzgießverfahren, im 3D-Druckverfahren und/oder durch zerspanende Technologien, insbesondere Fräsen, hergestellt.

Gemäß einer weiteren Lehre, der eigenständige Bedeutung zukommt, ist ein Verfahren unter Verwendung einer vorschlagsgemäßen Vorrichtung zur kontinuierlichen Vireninaktivierung während eines Proteinproduktionsprozesses, insbesondere eines Antikörperproduktionsprozesses, vorgesehen, wobei ein ein Zielprotein und vireninaktivierende Bedingungen beinhaltender Flüssigkeitsstrom 22 durch den Fluideinlass 20 in die Vorrichtung eingeleitet wird und zum Vorsehen einer Mindestverweildauer des Flüssigkeitsstroms 22 durch die Vorrichtung und anschließend durch den Fluidauslass 21 aus der Vorrichtung hinaus geleitet wird, vorzugsweise, dass die Vorrichtung beidseitig durchströmt werden kann, derart, dass der Flüssigkeitsstrom 22 alternativ durch den Fluidauslass 21 in die Vorrichtung eingeleitet und durch den Fluideinlass 20 aus der Vorrichtung ausgeleitet wird. Auf alle Ausführungen zur vorschlagsgemäßen Vorrichtung darf insoweit verwiesen werden.

Weiter kann das vorschlagsgemäße Verfahren in Kombination mit einem vorgeschalteten, vorzugsweise diskontinuierlichen oder kontinuierlichen, Chromatographieverfahren, insbesondere Affinitätschromatographie- und Ionenaustauschchromatographieverfahren, durchgeführt werden. Zusätzlich oder alternativ kann das vorschlagsgemäße Verfahren in Kombination mit einem vorgeschalteten, vorzugsweise diskontinuierlichen oder kontinuierlichen, Filtrationsverfahren, insbesondere mit Tangential Flow Filtrations-Verfahren, durchgeführt werden. Zusätzlich oder alternativ kann dem vorschlagsgemäßen Verfahren ein kontinuierliches Chromatographieverfahren und/oder Filtrationsverfahren nachgeschaltet sein.

Grundsätzlich kann die vorschlagsgemäße Vorrichtung sowie das vorschlagsgemäße Verfahren in Kombination mit sämtlichen Aufreinigungs-, Filtrations-, Chromatographie-, Separations-, Zentrifugations-, Konzentrierungs- und/oder Sedimentations-Verfahren oder sonstigen Verfahren, die dem Downstream-Prozess von Proteinprodukten zugeordnet werden können, verwendet werden.

Gemäß einer weiteren Lehre, der eigenständige Bedeutung zukommt, ist eine Verwendung einer vorschlagsgemäßen Vorrichtung zur Umsetzung eines Verfahrens zur kontinuierlichen Vireninaktivierung während eines Proteinproduktionsprozesses, insbesondere eines Antikörperproduktionsprozesses, beansprucht. Auf alle Ausführungen zur vorschlagsgemäßen Vorrichtung und zum vorschlagsgemäßen Verfahren darf insoweit verwiesen werden.

Gemäß einer weiteren Lehre, der eigenständige Bedeutung zukommt, wird die Verwendung eines vormontierten Verweilzeitprodukts 24 zur kontinuierlichen Vireninaktivierung während eines Proteinproduktionsprozesses, insbesondere eines Antikörperproduktionsprozesses, beansprucht, welches die Vorrichtung zur kontinuierlichen Vireninaktivierung, insbesondere teilweise oder vollständig, aufweist, vorzugsweise, welches zumindest die Hohlkörperhalteeinrichtung 1 und/oder den daran angeordneten Hohlkörper 2 aufweist, oder, welches zumindest die Tragstruktur 23, die Rollen 3, 4, 5, 6 und/oder das Unterstützungselement 9 aufweist. Auch hier gelten alle zuvor gemachten Ausführungen in vollem Umfang.

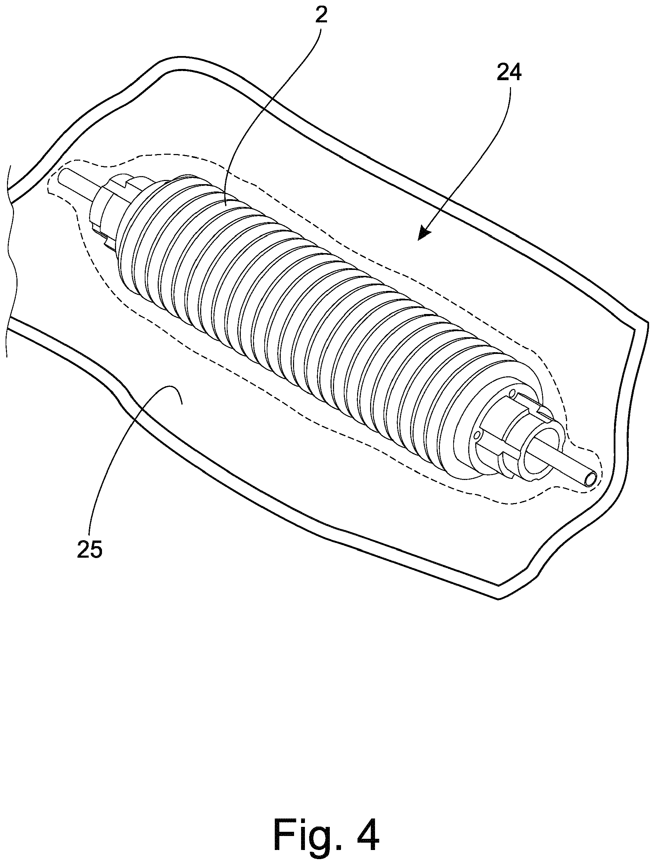

Hier und vorzugsweise kann das vormontierte Verweilzeitprodukt 24 als mindestens drei unterschiedliche, vormontierte Verweilzeitprodukte 24 ausgebildet sein. Das erste vormontierte Verweilzeitprodukt 24 weist vorzugsweise die gesamte vorschlagsgemäße Vorrichtung auf. Wie beispielhaft in Fig. 4 dargestellt, weist das zweite vormontierte Verweilzeitprodukt 24 vorzugsweise die Hohlkörperhalteeinrichtung 1 und den daran angeordneten Hohlkörper 2 auf. Das mindestens dritte vormontierte Verweilzeitprodukt 24 weist vorzugsweise die Tragstruktur 23, die Rollen 3, 4, 5, 6 und das Unterstützungselement 9 auf.

Das Verweilzeitprodukt 24 ist hier und vorzugsweise als Einwegkomponente ausgebildet. Vorzugsweise umfasst das vormontierte Verweilzeitprodukt 24 eine sterile Verpackung 25 zur sterilen Unterbringung des Verweilzeitprodukts 24 (Fig. 4). Ein entsprechender Austausch der Vorrichtung, insgesamt oder zumindest des jeweiligen Teils nach einmaliger Benutzung, gewährleistet die Sterilität und spart etwaige Reinigungsschritte nach Prozessende ein.

Gemäß einer weiteren Lehre, der eigenständige Bedeutung zukommt, ist eine Vorrichtung zur kontinuierlichen Vireninaktivierung während eines Proteinproduktionsprozesses, insbesondere eines Antikörperproduktionsprozesses beansprucht, wobei die Vorrichtung eine sich in einer Axialrichtung erstreckende Hohlkörperhalteeinrichtung 1 zum Halten eines länglichen, elastisch verformbaren Hohlkörpers 2, insbesondere eines Schlauchs, aufweist, wobei die Vorrichtung mindestens zwei sich in der Axialrichtung erstreckende Rollen 3, 4, insbesondere drei oder vier Rollen 3, 4, 5, 6, aufweist, mit denen im bestimmungsgemäß montierten Zustand der Vorrichtung der Hohlkörper 2 jeweils gequetscht wird und dadurch das innere Volumen 7 des Hohlkörpers 2 in fluidtechnisch voneinander getrennte Volumenabschnitte 8 unterteilt wird, wobei die kontinuierliche Vireninaktivierung in den Volumenabschnitten 8 des Hohlkörpers 2 durchgeführt wird.

Wesentlich bei der vorschlagsgemäßen Vorrichtung ist nun, dass die Vorrichtung zum Antrieb der Rollen 3, 4, 5, 6 mindestens eine Umlaufräderanordnung mit jeweils einem Sonnenrad 10, 11, mehreren mit diesem in Eingriff stehenden Planetenrädern 12 und einem mit diesen in Eingriff stehenden Hohlrad 13, 14 aufweist.

**Patentansprüche**

1. Vorrichtung zur kontinuierlichen Vireninaktivierung während eines Proteinproduktionsprozesses, insbesondere eines Antikörperproduktionsprozesses, wobei die Vorrichtung eine sich in einer Axialrichtung erstreckende Hohlkörperhichtung (1) zum Halten eines länglichen, elastisch verformbaren Hohlkörpers (2), insbesondere eines Schlauchs, aufweist, wobei die Vorrichtung mindestens zwei sich in der Axialrichtung erstreckende Rollen (3, 4), insbesondere drei oder vier Rollen (3, 4, 5, 6), aufweist, mit denen im bestimmungsgemäß montierten Zustand der Vorrichtung der Hohlkörper (2) jeweils gequetscht wird und dadurch das innere Volumen (7) des Hohlkörpers (2) in fluidtechnisch voneinander getrennte Volumenabschnitte (8) unterteilt wird, wobei die kontinuierliche Vireninaktivierung in den Volumenabschnitten (8) des Hohlkörpers (2) durchgeführt wird,
**dadurch gekennzeichnet,**
**dass** die Vorrichtung ein Unterstützungselement (9) aufweist, welches im bestimmungsgemäß montierten Zustand der Vorrichtung die Mantelflächen aller Rollen (3, 4, 5, 6) jeweils in mindestens einem Punkt abstützt.

2. Vorrichtung nach Anspruch 1, **dadurch gekennzeichnet, dass** die Vorrichtung zum Antrieb der Rollen (3, 4, 5, 6) mindestens eine Umlaufräderanordnung, vorzugsweise zwei axial voneinander beabstandete Umlaufräderanordnungen, mit jeweils einem Sonnenrad (10, 11), mehreren mit diesem in Eingriff stehenden Planetenrädern (12) und einem mit diesen in Eingriff stehenden Hohlrad (13, 14) aufweist, vorzugsweise, dass die Planetenräder (12) durch eine Zahnverbindung, vorzugsweise durch eine Evolventenverzahnung, mit einem zugeordneten der Sonnenräder (10, 11) und/oder der Hohlräder (13, 14) in kämmendem Eingriff stehen.

3. Vorrichtung nach Anspruch 2, **dadurch gekennzeichnet, dass** die Hohlkörperhalteeinrichtung (1) mindestens ein, insbesondere endseitig angeordnetes, Sonnenrad (10, 11), vorzugsweise zwei axial voneinander beabstandete, insbesondere endseitig angeordnete, Sonnenräder (10, 11), aufweist, das/die mit der Hohlkörperhalteeinrichtung (1) im Übrigen drehfest ist/sind, und/oder, dass die Rollen (3, 4, 5, 6) jeweils mindestens ein, insbesondere endseitig angeordnetes, Planetenrad (12), vorzugsweise zwei axial voneinander beabstandete, insbesondere endseitig angeordnete, Planetenräder (12), aufweisen, das/die mit der jeweiligen Rolle (3, 4, 5, 6) im Übrigen drehfest ist/sind, und/oder, dass das Unterstützungselement (9) mindestens ein, insbesondere endseitig angeordnetes, Hohlrad (13, 14), vorzugsweise zwei axial voneinander beabstandete, insbesondere endseitig angeordnete, Hohlräder (13, 14), aufweist, das/die mit dem Unterstützungselement (9) im Übrigen drehfest ist/sind.

4. Vorrichtung nach Anspruch 2 oder 3, **dadurch gekennzeichnet, dass** im bestimmungsgemäßen Betrieb der Vorrichtung das Hohlrad (13, 14) oder mindestens eines der Planetenräder (12) mindestens einer der Umlaufräderanordnungen motorisch angetrieben ist/sind, vorzugsweise, dass im bestimmungsgemä-ßen Betrieb der Vorrichtung die Sonnenräder (10, 11) starr angeordnet sind.

5. Vorrichtung nach einem der vorhergehenden Ansprüche, **dadurch gekennzeichnet, dass** das Unterstützungselement (9) hohlzylindrisch, ringförmig oder spiralförmig ausgestaltet ist, dass das Unterstützungselement (9) eine umlaufende, insbesondere zylindrische, ebene Innenoberfläche (15) aufweist, dass die Rollen (3, 4, 5, 6) eine zylindrische, ebene Außenoberfläche (16) aufweisen, dass sich im bestimmungsgemäßen Betrieb der Vorrichtung die Innenoberfläche (15) des Unterstützungselements (9) auf der Außenoberfläche der Rollen (16) abrollt, vorzugsweise, dass das Unterstützungselement (9) als axiale Verlängerung eines Hohlrads (13, 14) oder als axiale Verlängerung der beiden Hohlräder (13, 14) ausgestaltet ist, oder, dass das Unterstützungselement (9) die beiden Hohlräder (13, 14) axial miteinander verbindet.

6. Vorrichtung nach einem der vorhergehenden Ansprüche, **dadurch gekennzeichnet, dass** der Innendurchmesser des Unterstützungselements (9) auf den Außendurchmesser der Rollen (3, 4, 5, 6) derart abgestimmt ist, dass sich die Rollen (3, 4, 5, 6) und das Unterstützungselement (9) aneinander in einem Wälzpunkt abwälzen, und/oder, dass im bestimmungsgemäß montierten Zustand der Vorrichtung die Teilkreisdurchmesser der Planetenräder (12) der Rollen (3, 4, 5, 6) mit den Wälzkreisdurchmessern der Rollen (3, 4, 5, 6) im Übrigen identisch sind sowie die Teilkreisdurchmesser der Hohlräder (13, 14) mit dem Wälzkreisdurchmesser des Unterstützungselements (9) identisch sind.

7. Vorrichtung nach einem der vorhergehenden Ansprüche, **dadurch gekennzeichnet, dass** die Hohlkörperhalteeinrichtung (1) mindestens zwei, insbesondere mindestens drei oder vier, Durchführungsöffnungen (17) aufweist, welche die Innenoberfläche (18) der Hohlkörperhalteeinrichtung (1) mit der Außenoberfläche (19) der Hohlkörperhalteeinrichtung (1) verbinden, und dass der Hohlkörper (2) im bestimmungsgemäß montierten Zustand der Vorrichtung durch die Durchführungsöffnungen (17) hindurchgeführt ist, vorzugsweise, dass die Durchführungsöffnungen (17) die einzigen Verbindungen zwischen der Innenoberfläche (18) der Hohlkörperhalteeinrichtung (1) und der Außenoberfläche (19) der Hohlkörperhalteeinrichtung (1) bilden.

8. Vorrichtung nach einem der vorhergehenden Ansprüche, **dadurch gekennzeichnet, dass** der Hohlkörper (2) einen Fluideinlass (20) und einen Fluidauslass (21) aufweist, dass der Hohlkörper (2) auf die Hohlkörperhalteeinrichtung (1) aufgewickelt ist.

9. Vorrichtung nach Anspruch 8, **dadurch gekennzeichnet, dass** im bestimmungsgemäß montierten Zustand der Vorrichtung ein ein Zielprotein und vireninaktivierende Bedingungen beinhaltender Flüssigkeitsstrom (22) durch den Fluideinlass (20) in die Vorrichtung einleitbar ist und zum Vorsehen einer Mindestverweildauer des Flüssigkeitsstroms (22) durch die Vorrichtung hindurchleitbar und anschließend durch den Fluidauslass (21) aus der Vorrichtung ausleitbar ist.

10. Vorrichtung nach einem der vorhergehenden Ansprüche, **dadurch gekennzeichnet, dass** die Rollen (3, 4, 5, 6) bei gerader Anzahl am Außenumfang der Hohlkörperhalteeinrichtung (1) gegenüberliegend, insbesondere gleichmäßig über den Außenumfang der Hohlkörperhalteeinrichtung (1) verteilt, angeordnet und bei ungerader Anzahl gleichmäßig über den Außenumfang der Hohlkörperhalteeinrichtung (1) verteilt angeordnet sind.

11. Vorrichtung nach einem der vorhergehenden Ansprüche, **dadurch gekennzeichnet, dass** im bestimmungsgemäßen Betrieb der Vorrichtung, durch eine Relativbewegung der Rollen (3, 4, 5, 6) und der Hohlkörperhalteeinrichtung (1) zueinander, im Hohlkörper (2) eine Fluid-Förderung gemäß dem Prinzip einer Verdrängerpumpe derart erzeugt wird, dass die voneinander getrennten Volumenabschnitte (8) im Hohlkörper (2) im bestimmungsgemäßen Betrieb der Vorrichtung mit den voreingestellten vireninaktivierenden Bedingungen über die Prozesslaufzeit zueinander unvermischt transportiert werden.

12. Vorrichtung nach einem der vorhergehenden Ansprüche, **dadurch gekennzeichnet, dass** im bestimmungsgemäß montierten Zustand der Vorrichtung der Abstand zwischen Hohlkörperhalteeinrichtung (1) und Rollen (3, 4, 5, 6) höchstens der zweifachen Wandstärke des Hohlkörpers (2) entspricht.

13. Vorrichtung nach einem der vorhergehenden Ansprüche, **dadurch gekennzeichnet, dass** die Vorrichtung eine Tragstruktur (23) aufweist, dass alle Bestandteile der Vorrichtung direkt oder indirekt, und/oder zumindest die Hohlkörperhalteeinrichtung (1) direkt, an der Tragstruktur (23) angeordnet ist.

14. Vorrichtung nach einem der vorhergehenden Ansprüche, **dadurch gekennzeichnet, dass** alle Bestandteile der Vorrichtung, oder zumindest der Hohlkörper (2) und/oder die Hohlkörperhalteeinrichtung (1), als Einwegkomponenten ausgestaltet sind.

15. Vorrichtung nach einem der vorhergehenden Ansprüche, **dadurch gekennzeichnet, dass** die zur bestimmungsgemäßen Funktion mindestens notwendigen Bestandteile der Vorrichtung, insbesondere zumindest umfassend die Hohlkörperhalteeinrichtung (1) und den Hohlkörper (2), und/oder, zumindest umfassend die Tragstruktur (23), die Rollen (3, 4, 5, 6) und das Unterstützungselement (9), eine Baugruppe bilden, die als vormontierte oder einstückige Einheit ausgestaltet ist.

16. Vorrichtung nach einem der vorhergehenden Ansprüche, **dadurch gekennzeichnet, dass** die Tragstruktur (23), die Hohlkörperhalteeinrichtung (1), die Rollen (3, 4, 5, 6) und/oder das Unterstützungselement (9), insbesondere einzeln oder zusammen, im Kunststoff-Spritzgießverfahren, im 3D-Druckverfahren und/oder durch zerspanende Technologien, insbesondere Fräsen, hergestellt sind.

17. Verfahren zur kontinuierlichen Vireninaktivierung während eines Protein-produktionsprozesses, insbesondere eines Antikörperproduktionsprozesses, unter Verwendung einer Vorrichtung nach einem der vorhergehenden Ansprüche, wobei ein ein Zielprotein und vireninaktivierende Bedingungen beinhaltender Flüssigkeitsstrom (22) durch den Fluideinlass (20) in die Vorrichtung eingeleitet wird und zum Vorsehen einer Mindestverweildauer des Flüssigkeitsstroms (22) durch die Vorrichtung und anschließend durch den Fluidauslass (21) aus der Vorrichtung hinaus geleitet wird, vorzugsweise, dass die Vorrichtung beidseitig durchströmt werden kann, derart, dass der Flüssigkeitsstrom (22) alternativ durch den Fluidauslass (21) in die Vorrichtung eingeleitet und durch den Fluideinlass (20) aus der Vorrichtung ausgeleitet wird.

18. Verfahren nach Anspruch 17, **dadurch gekennzeichnet, dass** das vorschlagsgemäße Verfahren in Kombination mit einem vorgeschalteten, vorzugsweise diskontinuierlichen oder kontinuierlichen, Chromatographieverfahren und/oder Filtrationsverfahren, durchgeführt wird und/oder, dass dem vorschlagsgemäßen Verfahren ein kontinuierliches Chromatographieverfahren und/oder Filtrationsverfahren nachgeschaltet ist.

19. Verwendung einer Vorrichtung nach einem der Ansprüche 1 bis 16 zur Umsetzung eines Verfahrens zur kontinuierlichen Vireninaktivierung während eines Proteinproduktionsprozesses, insbesondere eines Antikörperproduktionsprozesses.

20. Verwendung eines vormontierten Verweilzeitprodukts (24) zur kontinuierlichen Vireninaktivierung während eines Proteinproduktionsprozesses, insbesondere eines Antikörperproduktionsprozesses, welches die Vorrichtung zur kontinuierlichen Vireninaktivierung, insbesondere teilweise oder vollständig, aufweist, vorzugsweise, welches zumindest die Hohlkörperhalteeinrichtung (1) und/oder den daran angeordneten Hohlkörper (2) aufweist, oder, welches zumindest die Tragstruktur (23), die Rollen (3, 4, 5, 6) und/oder das Unterstützungselement (9) aufweist.

21. Verwendung nach Anspruch 20, **dadurch gekennzeichnet, dass** das vormontierte Verweilzeitprodukt (24) als Einwegkomponente ausgebildet ist, vorzugsweise dass das vormontierte Verweilzeitprodukt (24) eine sterile Verpackung (25) zur sterilen Unterbringung des Verweilzeitprodukts (24) umfasst.

22. Vorrichtung zur kontinuierlichen Vireninaktivierung während eines Proteinproduktionsprozesses, insbesondere eines Antikörperproduktionsprozesses, wobei die Vorrichtung eine sich in einer Axialrichtung erstreckende Hohlkörperhalteeinrichtung (1) zum Halten eines länglichen, elastisch verformbaren Hohlkörpers (2), insbesondere eines Schlauchs, aufweist, wobei die Vorrichtung mindestens zwei sich in der Axialrichtung erstreckende Rollen (3, 4), insbesondere drei oder vier Rollen (3, 4, 5, 6), aufweist, mit denen im bestimmungsgemäß montierten Zustand der Vorrichtung der Hohlkörper (2) jeweils gequetscht wird und dadurch das innere Volumen (7) des Hohlkörpers (2) in fluidtechnisch voneinander getrennte Volumenabschnitte (8) unterteilt wird, wobei die kontinuierliche Vireninaktivierung in den Volumenabschnitten (8) des Hohlkörpers (2) durchgeführt wird,

**dadurch gekennzeichnet,**
**dass** die Vorrichtung zum Antrieb der Rollen (3, 4, 5, 6) mindestens eine Umlauffräderanordnung mit jeweils einem Sonnenrad (10, 11), mehreren mit diesem in Eingriff stehenden Planetenrädern (12) und einem mit diesen in Eingriff stehenden Hohlrad (13, 14) aufweist.

# Fig. 1

a)

b)

## Fig. 2

Fig. 3

Fig. 4

Europäisches
Patentamt
European
Patent Office
Office européen
des brevets

## EUROPÄISCHER RECHERCHENBERICHT

**Nummer der Anmeldung**

EP 23 18 2766

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2018/208447 A1 (EMD MILLIPORE CORP [US]) 15. November 2018 (2018-11-15) | 20 | INV. C12M1/12 |
| Y | * Seiten 1-6,8,9 * | 1-4, | A61L2/00 |
| | * Seiten 11-13,15 * | 7-14, | C12N7/00 |
| | * Seiten 16,18,21 * | 16-19, | C12M1/00 |
| | * Abbildungen 5,7,13b,13c * | 21,22 | B01D15/24 |
| A | | 5,6,15 | |
| | ----- | | |
| Y | US 2002/146338 A1 (MITTELSTEIN MICHAEL [US] ET AL) 10. Oktober 2002 (2002-10-10) | 1-4, 7-14, 16-19, 21,22 | |
| A | * Absätze [0002], [0011], [0035], [0037], [0041], [0059], [0060]; Abbildungen 3,4,11,14-17 * | 5,6,15 | |
| | ----- | | |
| Y | US 6 596 230 B1 (WOO LECON [US] ET AL) 22. Juli 2003 (2003-07-22) | 1-4, 7-14, 16-19, 21,22 | |
| A | * Spalten 1,3,4; Abbildungen 3,7,8 * | 5,6,15 | RECHERCHIERTE SACHGEBIETE (IPC) |
| | ----- | | |
| Y | DE 10 2010 000594 A1 (ULRICH GMBH & CO KG [DE]) 1. September 2011 (2011-09-01) | 1-4, 7-14, 16-19, 21,22 | C12M C07K A61L C12N |
| A | * Absätze [0001], [0006], [0009], [0012], [0023]; Abbildungen * | 5,6,15 | B01D |
| | ----- | | |
| Y | EP 1 862 672 A2 (KLAEMPFL FRANZ XAVER [DE]) 5. Dezember 2007 (2007-12-05) | 1-4, 7-14, 16-19, 21,22 | |
| A | * Absätze [0011], [0012], [0015]; Abbildungen * | 5,6,15 | |
| | ----- | | |
| | -/-- | | |

Der vorliegende Recherchenbericht wurde für alle Patentansprüche erstellt

| Recherchenort | Abschlußdatum der Recherche | Prüfer |
|---|---|---|
| Berlin | 13. Januar 2024 | Böhm, Ingo |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
................................................
& : Mitglied der gleichen Patentfamilie, übereinstimmendes
Dokument

EPO FORM 1503 03.82 (P04C03)

Europäisches
Patentamt

European
Patent Office

Office européen
des brevets

## EUROPÄISCHER RECHERCHENBERICHT

**Nummer der Anmeldung**

**EP 23 18 2766**

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2012/051147 A1 (ABBOTT LAB [US]; WANG CHEN [US]; HICKMAN ROBERT K [US]) 19. April 2012 (2012-04-19) * Absätze [0004], [0021] – [0028], [0059], [0060] * ----- | 1,17,19, 20,22 | |
| A | US 2021/380914 A1 (PARKER STEPHANIE [US] ET AL) 9. Dezember 2021 (2021-12-09) * Absätze [0004] – [0028], [0042] * ----- | 1,17,19, 20,22 | |
| A | CN 113 975 415 A (YU JIAJI) 28. Januar 2022 (2022-01-28) * Seite 1; Abbildung 5 * ----- | 1,17,19, 20,22 | |
| A | DE 28 48 706 A1 (KARTRIDG PAK CO) 26. Juli 1979 (1979-07-26) * Seiten 3,4,7,9; Abbildungen 5,8 * ----- | 1,17,19, 20,22 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| **Berlin** | **13. Januar 2024** | **Böhm, Ingo** |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................
& : Mitglied der gleichen Patentfamilie, übereinstimmendes Dokument

EPO FORM 1503 03.82 (P04C03)

**Seite 2 von 2**

## ANHANG ZUM EUROPÄISCHEN RECHERCHENBERICHT
## ÜBER DIE EUROPÄISCHE PATENTANMELDUNG NR.

EP 23 18 2766

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

**13-01-2024**

| Im Recherchenbericht angeführtes Patentdokument | Datum der Veröffentlichung | Mitglied(er) der Patentfamilie | Datum der Veröffentlichung |
|---|---|---|---|
| WO 2018208447 A1 | 15-11-2018 | CA 3062516 A1 | 15-11-2018 |
| | | CN 110891665 A | 17-03-2020 |
| | | EP 3621715 A1 | 18-03-2020 |
| | | JP 7117329 B2 | 12-08-2022 |
| | | JP 2020520350 A | 09-07-2020 |
| | | KR 20200004331 A | 13-01-2020 |
| | | TW 201900865 A | 01-01-2019 |
| | | US 2020172568 A1 | 04-06-2020 |
| | | WO 2018208447 A1 | 15-11-2018 |
| US 2002146338 A1 | 10-10-2002 | AU 2002244317 A1 | 08-10-2002 |
| | | US 2002146338 A1 | 10-10-2002 |
| | | WO 02077456 A2 | 03-10-2002 |
| US 6596230 B1 | 22-07-2003 | AT E460185 T1 | 15-03-2010 |
| | | AU 4142801 A | 07-08-2001 |
| | | AU 2001241428 B2 | 14-07-2005 |
| | | CA 2398247 A1 | 02-08-2001 |
| | | EP 1263481 A1 | 11-12-2002 |
| | | JP 4383705 B2 | 16-12-2009 |
| | | JP 2003520643 A | 08-07-2003 |
| | | JP 2009233346 A | 15-10-2009 |
| | | US 6596230 B1 | 22-07-2003 |
| | | WO 0154739 A1 | 02-08-2001 |
| DE 102010000594 A1 | 01-09-2011 | BR 112012022097 A2 | 23-08-2016 |
| | | CN 102782325 A | 14-11-2012 |
| | | DE 102010000594 A1 | 01-09-2011 |
| | | EP 2542781 A1 | 09-01-2013 |
| | | ES 2459308 T3 | 08-05-2014 |
| | | KR 20130025377 A | 11-03-2013 |
| | | RU 2012140061 A | 27-04-2014 |
| | | TW 201144605 A | 16-12-2011 |
| | | US 2013045121 A1 | 21-02-2013 |
| | | WO 2011107326 A1 | 09-09-2011 |
| EP 1862672 A2 | 05-12-2007 | DE 102006025009 A1 | 20-12-2007 |
| | | EP 1862672 A2 | 05-12-2007 |
| WO 2012051147 A1 | 19-04-2012 | AU 2011316730 A1 | 02-05-2013 |
| | | AU 2016201535 A1 | 31-03-2016 |
| | | BR 112013008738 A2 | 06-10-2015 |
| | | CA 2813747 A1 | 19-04-2012 |
| | | CN 103379949 A | 30-10-2013 |
| | | EP 2627425 A1 | 21-08-2013 |
| | | JP 6023715 B2 | 09-11-2016 |

EPO FORM P0461

Für nähere Einzelheiten zu diesem Anhang : siehe Amtsblatt des Europäischen Patentamts, Nr.12/82

**Seite 1 von 2**

**ANHANG ZUM EUROPÄISCHEN RECHERCHENBERICHT**
**ÜBER DIE EUROPÄISCHE PATENTANMELDUNG NR.**

EP 23 18 2766

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-01-2024

| Im Recherchenbericht angeführtes Patentdokument | Datum der Veröffentlichung | Mitglied(er) der Patentfamilie | Datum der Veröffentlichung |
|---|---|---|---|
| | | JP 2013539787 A | 28-10-2013 |
| | | KR 20130142128 A | 27-12-2013 |
| | | MX 344268 B | 09-12-2016 |
| | | NZ 608943 A | 24-04-2015 |
| | | RU 2013120948 A | 20-11-2014 |
| | | SG 189872 A1 | 28-06-2013 |
| | | SG 10201508401T A | 27-11-2015 |
| | | TW 201221641 A | 01-06-2012 |
| | | US 2012264920 A1 | 18-10-2012 |
| | | WO 2012051147 A1 | 19-04-2012 |
| US 2021380914 A1 | 09-12-2021 | AU 2019357965 A1 | 29-04-2021 |
| | | CA 3115253 A1 | 16-04-2020 |
| | | CN 113015787 A | 22-06-2021 |
| | | EP 3864131 A1 | 18-08-2021 |
| | | JP 2022504397 A | 13-01-2022 |
| | | JP 2023055813 A | 18-04-2023 |
| | | KR 20210073554 A | 18-06-2021 |
| | | MA 53856 A | 12-01-2022 |
| | | SG 11202103443X A | 28-05-2021 |
| | | US 2021380914 A1 | 09-12-2021 |
| | | WO 2020076681 A1 | 16-04-2020 |
| CN 113975415 A | 28-01-2022 | KEINE | |
| DE 2848706 A1 | 26-07-1979 | AU 3991078 A | 20-03-1980 |
| | | CA 1084830 A | 02-09-1980 |
| | | DE 2848706 A1 | 26-07-1979 |
| | | GB 2012710 A | 01-08-1979 |
| | | JP S54104998 A | 17-08-1979 |
| | | US 4211051 A | 08-07-1980 |

Für nähere Einzelheiten zu diesem Anhang : siehe Amtsblatt des Europäischen Patentamts, Nr.12/82

EPO FORM P0461

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018208447 A1